Europäisches Patentamt

European Patent Office

Office européen des brevets

(11) Publication number: **0 162 301 B1**

(12) EUROPEAN PATENT SPECIFICATION

(45) Date of publication of patent specification: **01.04.92**  (51) Int. Cl.⁵: **G01N 33/52**

(21) Application number: **85104791.0**

(22) Date of filing: **19.04.85**

The file contains technical information submitted after the application was filed and not included in this specification

(54) **Integral multilayer analytical element.**

(30) Priority: **19.04.84 JP 79159/84**

(43) Date of publication of application:
**27.11.85 Bulletin 85/48**

(45) Publication of the grant of the patent:
**01.04.92 Bulletin 92/14**

(84) Designated Contracting States:
**DE FR GB**

(56) References cited:
**EP-A- 0 044 775      EP-A- 0 097 952**
**EP-A- 0 101 945      EP-A- 0 103 901**
**GB-A- 2 085 159      US-A- 4 292 272**

(73) Proprietor: **FUJI PHOTO FILM CO., LTD.**
**210 Nakanuma Minami Ashigara-shi**
**Kanagawa 250-01(JP)**

(72) Inventor: **Arai, Fuminori, c/o Fuji Photo Film Co.Ltd.**
**3-11-46, Senzui**
**Asaka-shi, Saitama(JP)**
Inventor: **Katsuyama, Harumi, c/o Fuji Photo Film Co.Ltd.**
**3-11-46, Senzui**
**Asaka-shi, Saitama(JP)**
Inventor: **Osada, Chiaki, c/o Fuji Photo Film Co.Ltd.**
**3-11-46 Senzui**
**Asaka-shi, Saitama(JP)**
Inventor: **Yazawa, Kenichiro, c/o Fuji Photo Film Co:ltd.**
**3-11-46 Senzui**
**Asaka-shi, Saitama(JP)**

(74) Representative: **Kraus, Walter, Dr. et al**
**Patentanwälte Kraus, Weisert & Partner**
**Thomas-Wimmer-Ring 15**
**W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a dry-type integral multilayer analtyical element for determination of specific component (i.e., analyte) in a liquid sample. More particularly, this invention relates to an integral multilayer analytical element advantageously employable in an analysis of an aqueous liquid sample, especially in a clinical test using a body fluid as a sample.

Until now, as dry-type analytical elements, there have been proposed a number of integral multilayer analytical elements (hereinafter occasionally referred to simply as multilayer analytical elements) which comprise a transparent support, a water-absorbing reagent layer containing a hydrophilic polymer (binder) and a color-forming reagent superposed on the support and a porous spreading layer (hereinafter occasionally referred to simply as spreading layer) as the uppermost layer. The spreading layer of the multilayer analytical element functions to spread therein an aqueous liquid sample (e.g., blood such as whole blood, plasma or serum, lymph, saliva, spinal fluid, vaginal fluid, urine, drinking water, liquers, river water, water discharged from factory, etc.) which has been applied onto the surface of the spreading layer together with the components contained in the liquid sample in a lateral direction to supply the liquid sample into the water-absorbing layer (or a water-absorbing reagent-containing layer containing a hydrophilic polymer) at a uniform volume per unit surface area. This function is named a metering effect. As the porous spreading layers, there have been proposed non-fibrous porous spreading layers such as a non-fibrous anisotropic microporous medium layer, typically a membrane filter disclosed in Japanese Patent Provisional Publication No. 49(1974)-53888 (Patent Publication No. 53(1978)-21677) and United States Patent No. 3,992,158, three-dimensional lattice structure layer having continuous voids in which polymer microbeads are combined with each other through an adhesive agent resistant to swell with water disclosed in Japanese Patent Provisional Publication 55(1980)-90859 (United States Patent No. 4,258,001). These spreading layers are satisfactory in the spreading action for a variety of of water. However, if a whole blood sample is employed as the liquid sample, the solid components such as red blood cell are apt to plug the micro-voids of these spreading layers to disturb the spreading action. Further, even in the case that a plasma or serum containing a polymer component such as protein at a high concentration level is employed as the liquid sample, the polymer component is liable to plug the micro-voids to disturb the spreading action. In contrast, a porous spreading layers of woven fabric disclosed in Japanese Patent Provisional Publications No. 55(1980)-164356 (United States Patent No. 4,292,272) and No. 57(1982)-66359 can satisfactorily spread all of a whole blood, a plasma, and a serum. Further, the spreading layer of woven fabric is very advantageous from the viewpoint of the easy handling for manufacture of an integral multilayer analytical element, as well as from the viewpoint of the superior mechanical strength of the element.

GB-A-2 085 159 discloses a multilayer chemical analysis element. Fabrics used as spreading layers are described in this document. On page 7, lines 112-118, the impregnation of such fabrics with a surface active agent and a hydrophilic polymer is indicated. It is pointed out that the hydrophilic polymers disclosed in this reference are gelatin and polyvinyl alcohol. No mention is given with respect to the hydrophilic cellulose derivative.

It has been now found, however, that the spreading layer of woven fabric (hereinafter referred to as "woven fabric spreading layer") is hardly adjustable to vary the spread area (or spreadable area) from the inherently-given spread area, maintaining the necessary metering effect. For instance, if an analyte content in an aqueous liquid sample (hereinafter occasionally referred to as simply "a liquid sample" or "test liquid") is small, it is desirable to decrease the spread area (i.e., area in which the liquid sample is spread). In the case that the woven fabric spreading layer is employed as the spreading layer, the decrease of the spread area is difficultly attained or is only attained with reduce the metering action.

An object of the invention is to provide an integral multilayer analytical element having a porous spreading layer the spread area of which is easily adjustable over a wide range without reducing the spreading action (metering action).

Another object of the invention is to provide an integral multilayer analytical element which is employable to quantitatively analyze a micro-amount of substance originating from a living body such as uric acid, creatinine, ammonia, and various enzymes (active value).

A further object of the invention is to provide an integral multilayer analytical element employing blood as the aqueous liquid sample the use of which facilitates determination of a relationship between a value measured on a whole blood sample and a value measured on a plasma or serum sample and further facilitates conversion between the measured values.

A still further object of the invention is to provide an integral analytical element which makes it possible to determine a hematocrit value and a hemoglobin concentration in a whole blood sample.

The present invention provides an integral multilayer analytical element which comprises:

a porous spreading layer of woven fabric or knitted fabric, which contains a hydrophilic cellulose derivative and a nonionic surfactant having an HLB value of not less than 10, or a combination of such surfactants,

a water-absorbing layer containing a hydrophilic polymer, and

a light-transmissive, water-impermeable support.

Thus, the principal characteristic feature of the invention resides in the use of a spreading layer of woven fabric or knitted fabric and further in the incorporation of a hydrophilic cellulose derivative and a nonionic surfactant having an HLB value of not less than 10.

As the light-transmissive, water-impermeable support of the integral multilayer analytical element of the invention, there can be mentioned light-transmissive, water-impermeable supports employed in the multilayer analytical elements disclosed in the aforementioned patent publications. A representative support is a transparent polymer support having a thickness of 50 $\mu$m to 1 mm, preferably 80 $\mu$m to 300 $\mu$m. Examples of the polymer include polyethylene terephthalate, polycarbonate of bisphenol A, polystyrene, and cellulose esters (e.g., cellulose diacetate, cellulose triacetate, cellulose acetate propionate). If necessary, a subbing layer or an adhesive layer (which is known as such by the aforementioned patent publications) can be be provided to the surface of support so as to strengthen the bonding between the support and the below-mentioned water-absorbing layer or reagent-containing layer.

The water-absorbing layer comprises as a main component a hydrophilic polymer which swells upon absorbing water, and accordingly is capable of absorbing a water reaching the surface thereof or permeating therein. The hydrophilic polymer can be chosen from natural or synthetic hydrophilic polymer having a swelling index of 150 to 2,000 %, preferably 250 to 1,500 % (water at 30°C). Examples of the hydrophilic polymer include gelatins (e.g., acid-treated gelatin, deionized gelatin, etc.), gelatin derivatives (e.g., phthalated gelatin, hydroxyacrylate-grafted gelatin, etc.), agarose, pulluran, pulluran derivatives, polyacrylamide, polyvinyl alcohol, and polyvinylpyrrolidone. The water-absorbing layer generally has a thickness of 1 to 100 $\mu$m, preferably 3 to 50 $\mu$m, most preferably 5 to 30 $\mu$m, under dry condition. Preferably, the water-absorbing layer is transparent. The water-absorbing layer can contain a known mordant, basic polymer and/or acidic polymer.

The water-absorbing or water-permeable reagent-containing layer (occasionally referred to simply as "reagent layer") is a substantially nonporous water-absorbing layer or a microporous water-permeable layer which contains a hydrophilic polymer (binder) and can contain at least one reagent producing a detectable change upon reaction with an analyte (a component to be determined) in a liquid sample. The detectable change generally means a change which is detectable by a photometric measurement. Examples of the detectable change include color change, color formation, emission of fluorescence, change of absorbance in the ultraviolet reagion, and production of turbidity.

The reagent to be incorporated into the reagent layer is chosen according to the combination of an analyte in a liquid sample and a reaction system chosen for performing the analysis of the analyte. In the case that two or more reagents participate in the reaction, these reagents can be incorporated into one reagent layer, for instance, in the form of a mixture, and otherwise these reagents can be incorporated independently into plural layers. Examples of the reagent to be incorporated into the reagent layer include reagents containing enzyme(s) disclosed in the aforementioned patent publications, other known analytical reagent(s) and reagents employed for biochemical tests in clinical diagnosis.

The hydrophilic polymer employed for the formation of the substantially nonporous water-absorbing reagent layer serves as a medium for substantially uniformly dissolving or dispersing therein the reagent or reagent composition. Moreover, the hydrophilic polymer serves for absorbing the water of the liquid sample so as to supply the analyte together with water into the reagent layer. The hydrophilic polymer employed for this purpose can be selected from the water-absorbing hydrophilic polymers described hereinbefore with respect to the water-absorbing layer. The substantially nonporous reagent layer generally has a thickness of 3 to 50 $\mu$m, preferably 5 to 30 $\mu$m, under dry condition. Preferably, the reagent layer is transparent.

The microporous water-permeable reagent layer is a microporous layer containing a reagent or a reagent composition within a microporous matrix layer formed by a solid particulate and a hydrophilic polymer (binder). In more detail, the microporous matrix layer is formed of binding microporous particles or nonporous particles with the hydrophilic polymer binder to give a microporous continuous void structure.

Examples of the microporous particles and nonporous particles employed for the formation of the microporous reagent layer include cellulose microparticles such as microcrystalline cellulose, cellulose micropowder, and cellulose micropartilces; silicon dioxide microparticles such as silica and diatomaceous earth; polymer beads, glass beads, and ceramic beads. The hydrophilic polymer can be chosen from the water-absorbing hydrophilic polyer described hereinbefore with respect to the water-absorbing layer. Also employable is an aqueous latex of a copolymer containing a hydrophilic group as a repeating constitutional unit in an amount of not less than 2 %, as disclosed in Japanese Patent Provisional Publication No. 59-(1984)-145965. The microporous reagent layer generally has a thickness of 7 to 50 μm, preferably 10 to 30 μm, under dry condition.

The reagent layer can contain a known pH buffer composition, a polymer pH buffer, a basic polymer, an acidic polymer, a polymer mordant, etc.

The water-absorbing layer or the substantially non-porous reagent layer can be formed on the aforementioned light-transmissive, water-impermeable support according to a known coating method. The microporous reagent layer can be formed on the support according to the method disclosed in Japanese Patent Application No. 57(1982)-227980 or Japanese Patent Provisional Pubulication No. 59(1984)-145965. If necessary, the surface of the support can be processed according to the known phyico-chemical or chemical activation method, or provided with a transparent subbing layer (e.g, gelatin subbing layer), so that the bonding between the support and the water-absorbing layer or between the support and the reagent layer can be strengthened. Moreover, an water-absorbing layer can be provided between the support and the reagent layer. Particularly, the provision of a water-absorbing layer between the support and the reagent layer is preferred in the case that the reagent layer is a microporous reagent layer.

On the reagent layer or water-absorbing layer, a light-shielding layer can be formed. The light-shielding layer is a water-passable or water-permeable layer in which light-shielding microparticles or a light-shielding and light-reflective microparticles is dispered in a small amount of a film-forming hydrophilic polymer. The light-shielding layer shields a color of an aqueous liquid sample, particularly the red color of hemoglobin contained in a whole blood sample, applied to the below-described porous spreading layer in the reflection measurement of the detectable change taking place in the reagent layer or water-absorbing layer on he light-transmissive support side. Further, the light-shielding layer can function as a light-reflecting layer and a background layer.

Examples of the light-shielding and light-reflecting particles include titanium dioxide microparticles (e.g., titanium dioxide microcrystalline particles of the rutile type, anatase type or brookite type, having particle size of 0.1 to 1.2 μm), barium sulfate microparticles, and aluminum microparticles or micro-flakes. Examples of the light-shielding microparticles include carbon black, gas black and carbon microbeads. Among these microparticles, titanium dioxide microparticles and barium sulfate microparticles are preferred. As the film-forming hydrophilic polymer, there can be mentioned the hydrophilic polymer stated hereinbefore with respect to the water-absorbing layer, and weak water-hydrophilic regenerated cellulose and cellulose acetate. Among these polymers, gelatin, gelatin derivatives, and polyacrylamide are preferred. The gelatin and gelatin derivatives can be employed in combination with a known hardening agent (i.e., cross-linking agent).

The light-shielding layer can be formed on the reagent layer or water-absorbing layer by coating an aqueous dispersion containing light-shielding microparticles and a hydrophilic polymer and then drying the coated layer in a known manner. The volume ratio of the hydrophilic polymer to the light-shielding microparticles under dry condition ranges generally from 2.5 to 7.5, preferably from 3.0 to 6.5, to 10 of the microparticles. In the case that the light-shielding microparticles are titanium dioxide microparticles, the hydrophilic polymer is incorporated in a weight ratio of generally 0.6 to 1.8, preferably 0.8 to 1.5, to 10 of the titanium dioxide. The light-shielding layer has a thickness of 3 to 30 μm, preferably 5 to 20 μm, under dry condition.

On the reagent layer, water-absorbing layer or light-shielding layer, an adhesive layer can be provided to assist the provision of the below-mentioned porous spreading layer. Especially in the case that the light-shielding layer is arranged, an adhesive layer is preferably incorporated. The adhesive layer can be composed mainly of a hydrophilic polymer which can be united with the porous spreading layer under such conditions that the adhesive layer is wetted or swollen with water. As the hydrophilic polymer, there can be mentioned the hydrophilic polymers described hereinbefore with respect to the water-absorbing layer. Among these hydrophilic polymers, gelatin, gelatin derivatives and polyacrylamide are preferred. The adhesive layer has a thickness of 0.5 to 20 μm, preferably 1 to 10 μm, under dry condition. The adhesive layer can contain a surfactant. The surfactant preferably is a nonionic surfactant, especially a nonionic surfactant having a chain structure of 8 to 15 oxyethylene or oxypropylene groups.

The adhesive layer can be formed on the reagent layer, water-absorbing layer or light-shielding layer by coating an aqueous solution containing a hydrophilic polymer and an optionally added surfactant in a known manner.

On the water-absorbing layer or reagent layer is a provided a porous spreading layer of woven fabric (or woven fabric-like material) or knitted fabric (or knitted fabric-like material) directly or via a light-shielding layer (or light-reflecting layer) and an adhesive layer. The porous spreading layer is capable of spreading laterally therein an aqueous liquid sample which has been applied onto the upper surface thereof (surface

4

on the side farthermost from the light-transmissive support) to supply the components of the liquid sample into the water-absorbing layer or water-absorbing or water-permeable reagent layer (containing a hydrophilic polymer) at uniform volume per unit surface area, that is, a spreading action or metering action. The porous spreading layer also functions as a filter layer to remove insoluble material in an aqueous liquid sample (e.g., red blood cell in a whole blood sample) which is apt to disturb the analysis.

As the woven fabric (including woven fabric-like material), there can be mentioned a variety of woven fabric disclosed in Japanese Patent Provisional Publications No. 55(1980)-164356 and No. 57(1982)-66359. Among the woven fabrics, a plain weave cloth fabricated with warp and weft is preferred, and particularly preferred are sheeting cloth, shirting cloth, broad cloth and popline cloth. The yarns can be of any material mentioned hereinafter with respect to the knitted fabric. The yarn can be a filament yarn or spun yarn. The spun yarn is preferred. The yarn of the woven fabric generally is in the range of 20 S to 150 S (in terms of cotton spun yarn count), preferably 40 S to 120 S, or in the range of 35 D to 300 D (in terms of silk yarn denier), preferably 35 D to 90 D. The thickness of the woven fabric ranges generally from 100 $\mu$m to 500 $\mu$m, preferably 120 $\mu$m to 350 $\mu$m. The void ratio of the woven fabric ranges generally from 40 % to 90 %, preferably from 50 % to 85 %.

As the knitted fabric (including knitted fabric-like material), there can be mentioned a wide variety of knitted materials such as warp knitting and weft knitting. Examples of the warp knitting include single atlas stitch fabric, single tricot stitch fabric, double tricot stitch fabric, milanese stitch fabric and raschel stitch fabric. Examples of the weft knitting include plain stitch fabric, pearl stitch fabric, rib stitch fabric and interlock stitch fabric. The knitted fabric is generally made of natural yarns such as cotton yarn, silk yarn and wool yarn; and yarns (including filaments) of regenerated cellulose such as viscous rayon and cuprammonium rayon, semisynthetic organic polymers such as cellulose diacetate and cellulose triacetate, and synthetic organic polymers such as polyamide (e.g., various nylon), acetallized polyvinyl alcohol (e.g., vinylon), polyacrylnitrile, polyethylene terephthalate, polyethylene, polypropylene and polyurethane, and mixtures of natural yarns and regenerated yarns, semisynthethic organic polymer yarns and synthetic organic polymer yarns. The yarn can be a filament yarn or spun yarn. The spun yarn is preferred. The yarn of the knitted fabric generally is in the range of 40 S to 150 S (in terms of cotton spun yarn count), preferably 60 S to 120 S, or in the range of 35 D to 130 D (in terms of silk yarn denier), preferably 35 D to 90 D. The gauge number adopted for manufacturing the knitted fabric generally ranges from 20 to 50. The thickness of the knitted fabric ranges generally from 100 $\mu$m to 600 $\mu$m, preferably 150 $\mu$m to 400 $\mu$m. The void ratio of the knitted fabric ranges generally from 40 % to 90 %, preferably from 50 % to 85 %. Among the warp knittings, single tricot stitch fabric, double tricot stitch fabric, milanese stitch fabric and raschel stitch fabric are preferred, because these are hardly stretchable in the warp direction, are easily handled in the below-described lamination process of the knitted fabric spreading layer, and hardly become unlaced.

The woven fabric or knitted fabric to be employed for the formation of the knitted fabric spreading layer is substantially free from oil and fat which is apt to attach or supplied to the fabric in the course of the yarn manufacture or fabric manufacture and removed by a defatting (deoiling) process such as washing with water. The woven fabric or knitted fabric can be made hydrophilic, for instance, by a physical activation treatment (preferably, glow-discharge treatment or corona-discharge treatment) applied to at least one side of the woven fabric or knitted fabric as disclosed in Japanese Patent Provisional Publication No. 57(1982)-66359; or by a surfactant impregnation treatment (preferably, a nonionic surfactant impregnation treatment), or a hydrophilic polymer impregnation treatment as disclosed in Japanese Patent Provisional Publications No. 55(1980)-164356 and No. 57(1982)-66359; or an optional combination of two or more these treatments. Thus treated hydrophilic woven fabric or knitted fabric is satisfactory in the adhesion to the underlayer.

The woven fabric or knitted fabric can be laminated under adhesion on the reagent layer, water-absorbing layer or adhesive layer in the manner as disclosed in Japanese Patent Provisional Publications No. 55(1980)-164356 and No. 57(1982)-66359. In more detail, a woven fabric or knitted fabric is applied under substantially uniform light pressure to the undried reagent layer, water-absorbing layer or adhesive layer which is provided just after the coating procedure is complete. Alternatively, the dried reagent layer, water-absorbing layer or adhesive layer is swollen by supplying water substantially uniformly into the layer, and the woven fabric or knitted fabric is then applied onto the swollen layer in the same manner as above. Thus, the woven fabric or knitted fabric is combined with other layers to give the integral structure. In the case of employing gelatin or a gelatin derivative as the hydrophilic polymer binder in the reagent layer, water-absorbing layer or adhesive layer, the woven fabric or knitted fabric can be applied onto the undried gelatin (or gelatin derivative) layer still under a gelation condition just after the coating is complete, so as to give the desired integral structure.

Examples of the hydrophilic cellulose derivative incorporatable into the porous spreading layer of the integral multilayer analytical element of the present invention include cellulose ethers a portion or whole of the hydroxyl groups of which are substituted with lower alkyl groups containing 1 - 3 carbon atoms or hydroxyl-substituted lower alkyl groups containing 1 - 4 carbon atoms. Among these cellulose ethers, water-soluble cellulose ethers are preferred. Examples of the cellulose ethers include methylcellulose, ethylcellulose, hydroxyethylcellulose, hydroxypropylmethylcellulose, and hydroxybutylmethylcellulose. The hydrophilic cellulose derivatiive is generally incorporated into the porous spreading layer in the amount of 0.5 to 15 g. (preferably 0.7 to 10 g.) per 1 m$^2$ of the porous spreading layer.

As the nonionic surfactant having an HLB value (Hydrophile-Lipophile-Balance defined in J. Soc. Cosmet. Chem., 1, 311(1949) and "Kagaku(Science)" 23, 546(1953)) of not less than 10, there can be mentioned ethyleneoxide addition compounds of polyhydric alcohol esters (condensates), polyethylene glycol monoesters, polyethylene glycol diesters, ethyleneoxide addition compounds of of higher alcohols (condensates), ethyleneoxide addition compounds of alkylphenols (condensates), and alkanolamides of higher fatty acids. The nonionic surfactants having an HLB value of not less than 10 can be employed in combination. Moreover, the nonionic surfactants having an HLB value of not less than 10 can be employed in combination with other surfactants such as a nonionic surfactant having an HLB value of less than 10. In the latter case, the HLB value can be suitably adjusted.

Examples of the nonionic surfactant having an HLB balue of not less than 10 are given below together with HLB values thereof. It should be understood that the nonionic surfactant of the invention is not restricted to the below-listed nonionic surfactants.

| Nonionic Surfactant | | HLB Value |
|---|---|---|
| POE (20) | Sorbitan monooleate | 15.0 |
| POE (10) | Sorbitan monooleate | 13.5 |
| POE (4) | Sorbitan tristearate | 10.5 |
| POE (4) | Trioleate | 11.0 |
| POE (30) | Stearate | 16.0 |
| POE (40) | Stearate | 16.9 |
| POE (100) | Stearate | 18.8 |
| PEG (400) | Monostearate | 11.6 |
| PEG (400) | Monolaurate | 13.1 |
| PEG (1000) | Dilaurate | 14.1 |
| PEG (1540) | Distearate | 14.8 |
| EO (6) | Condensate of lauryl alcohol | 11.8 |
| EO (10) | Condensate of lauryl alcohol | 14.1 |
| EO (30) | Condensate of lauryl alcohol | 17.4 |
| EO (20) | Condensate of oleyl alcohol | 15.3 |
| EO (20) | Condensate of cetyl alcohol | 15.7 |
| POE (10) | Octylphenyl ether | 13.5 |
| POE (15) | Octylphenyl ether | 15.1 |
| POE (30) | Octylphenyl ether | 17.4 |
| POE (12) | Nonylphenyl ether | 14.1 |
| POE (20) | Nonylphenyl ether | 16.0 |
| Triethanolamine oleate | | 12.0 |

Remark)    POE: polyethyleneoxide

PEG: polyethylene glycol

EO: ethylene oxide

Numeral put in parentheses means a number of condensed ethyleneoxide units.

The nonionic surfactant is incorporated into the porous spreading layer generally in the range of 0.1 to 3 g., preferably 0.2 to 2 g., per 1 m$^2$.

The hydrophilic cellulose derivative and nonionic surfactant having an HLB value of not less than 10 can be incorporated into the porous spreading layer in any of the manners described below. Both are mixed to give an aqueous solution, and the solution is substantially uniformly coated or sprayed over the porous spreading layer and dried. Alternatively, a woven fabric or knitted fabric is dipped into the solution containing both components and then laminated under dry or semidry condition on the water-absorbing layer, reagent layer or adhesive layer to give a composite structure. The hydrophilic cellulose derivative is initially introduced into the porous spreading layer in the above-mentioned manner, and then the nonionic surfactant in an organic solvent (e.g., acetone, etc.) is introduced into the spreading layer in the same manner. The order of the introduction of the components stated above can be reversed. Further, both components can be introduced into the porous spreading layer similtaneously with introduction of other reagents.

The reagent composition or a portion or component thereof can be incorporated into the porous spreading layer. Especially in the case that the reagent composition includes a polymer substance, typically enzyme, or a dissociating agent for analyte conjugated with protein, said reagent component is almost preferably incorporated into the porous spreading layer. The incorporation of the reagent composition or a portion or component thereof into the spreading layer can be done in the manner as disclosed in Research Disclosure #12626 (October 1974), Japanese Patent Provisional Publication No. 50(1975)-137192 (corresponding to United States Patent No. 3,983,002) and Japanese Patent Provisional Publication No. 57-(1982)-208997 (corresponding to United States Patent No. 4,452,887), for enzyme; Japanese Patent Provisional Publication No. 57(1982)-208998, for substrate of enzyme; Japanese Patent Provisional Publication No. 59(1984)-171864, for a dissociating agent for analyte associated with protein; Japanese Patent Provisional Publication No. 59(1984)-49854 (Example 7), for other components.

The integral multilayer analytical element of the present invention can be provided, if necessary, with a detection layer or a mordant layer as disclosed in Japanese Patent Provisional Publication No. 51(1976)-40191 (corresponding to United States Patent No. 4,042,335), Japanese Patent Provisional Publication No. 53(1978)-131089 (corresponding to United States Patent No. 4,144,306); a migration-inhibition layer as disclosed in Japanese Patent Provisional Publication No. 54(1979)-29700 (corresponding to United States Patent No. 4,166,093); an intermediate layer as disclosed in Japanese Patent Provisional Publication No. 51(1976)-40191; a reagent-containing microparticle-dispersing layer as disclosed in Japanese Patent Provisional Publication No. 56(1981)-8549 (corresponding to United States Patent No. 4,356,149); a uniform thin barrier layer of a specific hydrophobic polymer as disclosed in Japanese Patent Provisional Publication No. 52(1977)-3488 (corresponding to United States Patent (Re) No. 30,267); an air barrier layer disclosed in Japanese Patent Provisional Publication No. 58(1983)-77661 and Japanese Patent Application No. 58(1983)-153822; and a gas-permeable adhesive intermediate layer as disclosed in Japanese Patent Application No. 58(1983)-128759.

The integral multilayer analytical element of the present invention can be cut to give a small square chip (length of one side: 15 to 30 mm) or round sheet of almost same size and encased in a slide frame, to give an analytical slide as disclosed in Japanese Patent Provisional Publication No. 57(1982)-63452, Japanese Patent Provisional Publication No. 54(1979)-156079 (corresponding to United States Patent No. 4,169,751), Japanese Utility Model Provisional Publication No. 56(1981)-142454 (corresponding to United States Patent No. 4,387,990), Japanese Utility Model Provisional Publication No. 58(1983)-32350, and Japanese Patent Provisional PCT Publication No. 58(1983)-501144 (corresponding to WO 83/00391). The analytical element in the form of the analytical slide is advantageous in all aspects such as manufacture, packing, transfer, storage and measurement procedure.

The integral multilayer analytical element of the invention can be used in the manner as disclosed in the aforementioned patent publications. For instance, an aqueous liquid sample in the amount of 5 to 30 $\mu\ell$, preferably 8 to 15 $\mu\ell$, is spotted on the porous spreading layer, and if necessary (particularly in the case of employing an enzyme as a component of the reagent composition ) the analytical element is then subjected to incubation at a substantially constant temperature in the range of 20 to 45ºC. The element is photometrically measured through reflection photometry on the light-transmissive support side to detect a detectable change such as color change or color formation shown in the element. The measured value was colorimetrically treated to quantitatively determine the analyte in the liquid sample. In the case of using an integral multilayer analytical element having a water-absorbing layer and a woven fabric or knitted fabric spreading layer on a light-transmissive, water-impermeable support, a hematocrit value or hemoglobin concentration in a blood can be determined in the same manner as in the case of using an integral multilayer analytical element as disclosed in Japanese Patent Provisional Publication No. 56(1981)-96245 (United States Patent No. 4,340,565) and Japanese Patent Provisional Publication No. 56(1981)-97872 (United States Patent No. 4,337,222).

The integral multilayer analytical element of the present invention can be prepared in the form of having a woven fabric or knitted fabric porous layer for supplying an aqueous liquid sample via a fixed area defined by a certain shape thereof (i.e., patch) which is provided as the uppermost layer, as disclosed in Japanese Utility Model Provisional Publication No. 57(1982)-42951.

The present invention is further illustrated by the following examples and comparison examples.

## Example 1

An aqueous gelatin (alkali-treated and deionized) solution was coated on a gelatin subbing layer of a colorless, transparent polyethylene terephthalate (PET) film (support, thickness: 180 $\mu$m) to give a layer of thickness of 20 $\mu$m (dry basis). The coated layer was then dried to give a water-absorbing layer.

One surface of a plain weave broad cloth (thickness: 140 $\mu$m) of polyethylene terephthalate (PET) spun yarn (count: equivalent to approx. cotton count 100 S) was processed by glow discharge for 60 s (1.6 kW/m$^2$, oxygen concentration was controlled under reduced pressure). The processed cloth was then treated with an acetone solution containing 1 wt% of hydroxypropylcellulose (methoxy group content: 28 - 30 %, hydroxypropoxy group content: 7 - 12 %, viscosity: 50 cps at 20ºC in 2% aqueous solution) and 1 wt% of polyoxyethylene octylphenyl ether (number of added ethylene oxides : 30 moles, HLB value: 17.4). Thus, the cloth was impregnated with the nonionic surfactant and hydrophilic cellulose derivative.

On the water-absorbing layer having been almost uniformly wetted with water was placed the treated broad cloth to face the glow discharge-processed surface with the water-absorbing layer. The composite was then passed between pressure rollers to uniformly combine the plain weave broad cloth with the water-absorbing layer. Thus, an integral multilayer analytical element having a woven fabric spreading layer was prepared.

Comparison Examples 1 & 2

The procedures of Example 1 were repeated except that the plain weave broad cloth was impregnated with the nonioic surfactant only (Comparison Example 1) or with the hydrophilic cellulose derivative only (Comparison Example 2). Thus, two integral multilayer analytical elements were produced. The spread area was measured in the same manner as in Example 1. The results are set forth in Table 1.

On the woven fabric spreading layer of thus produced integral multilayer analytical element (prepared in Example 1 or Comparison Example 1 or 2) was spotted 10 $\mu\ell$ of human plasma. The period of time for spreading and the spread area were measured. The results are set forth in Table 1.

Table 1

| Analytical Element | Spread Area | Period for Spreading | Condition of Spreading |
|---|---|---|---|
| Example 1 | 120 mm$^2$ | 10 s | Uniform |
| Com. Ex. 1 | 250 mm$^2$ | 10 s | Uniform |
| Com. Ex. 2 | 150 mm$^2$ | 25 s | Non-uniform |

The integral multilayer analtyical element of the invention was favorable in the spread area, period of time needed for the completion of the spreading action and condition of the spreading action. In contrast, the multilayer analytical element of Comparison Example 1 (in which the woven fabric spreading layer was impregnated with only a nonionic surfactant of an HLB value 17.4) showed an excessively large spread area approx. twice as large as a preferred spread area, while the multilayer analytical element of Comparison Example 2 (in which the woven fabric spreading layer was impregnated with only a hydrophilic cellulose derivative) showed a unsuitable spreading condition.

Example 2

The procedures of EXample 1 were repeated except that the composition of the solution for treating the plain weave broad cloth was replaced with the following composition.

| Plain Weave Broad Cloth Treating Solution | |
|---|---|
| Methylcellulose [viscosity 5 Pa•s (50 cps)] at 20°C, measured on 2 % aqueous solution) | 2 g. |
| Polyoxyethylene octylphenyl ether (containing 15(average) oxyethylene units, HLB: 15.1) | 1 g. |
| Water | 98 g. |

Thus, an integral multilayer analytical element was prepared.

On the woven fabric spreading layer of the multilayer analytical element was spotted a human plasma in an amount of from 2 $\mu\ell$ to 12 $\mu\ell$. A relationship between the spotted amount (x $\mu\ell$) and the spread area (y mm$^2$) is expressed by the following linear equation:

y = 15.67x + 1.81.

The corelation coefficient (r) is 0.9995.

Example 3

The procedures of EXample 1 were repeated except that the composition of the solution for treating the plain weave broad cloth was replaced with the following composition using surfactants having different HLB values.

| Plain Weave Broad Cloth Treating Solution | |
|---|---|
| Methylcellulose [viscosity 5 Pa•s (50 cps) at 20°C], measured on 2 % aqueous solution) | 2.5 g. |
| Nonionic surfactant (indicated in Table 2) | 1 g. |
| Water | 98 g. |

Thus, an integral multilayer analytical element was prepared.

On the woven fabric spreading layer of the multilayer analytical element was spotted 7% aqueous albumin solution in the amount of from 10 $\mu\ell$. The spread area was measured. The results are set forth in Table 2.

Table 2

| HLB Value | Nonionic Surfactant | Spread Area |
|---|---|---|
| 4.5 | PrG monolaurate | 140 mm$^2$ |
| 6.1 | DiEG monostearate | 125 mm$^2$ |
| 7.8 | PEG(200) stearate monoester | 115 mm$^2$ |
| 10.5 | POE(4)Srb tristearate | 85 mm$^2$ |
| 11.8 | EO(6) condensate of lauryl alcohol | 67 mm$^2$ |
| 15.1 | POE(15) OctPh ether | 43 mm$^2$ |
| 16.0 | POE(20) NonPh ether | 60 mm$^2$ |
| 17.0 (*) | POE(30) OctPh ether 82.6 % POE(15) OctPh ether 17.4 % | 50 mm$^2$ |
| 17.5 (*) | POE(100) stearate 54 % POE(30) OctPh ether 46 % | 42 mm$^2$ |

Remarks    PrG: propylene glycol,   EG: ethylene glycol, Srb: sorbitan,   OctPh: octylphenyl, NonPh: nonyl-phenyl

Other expressions are the same as in the aforemenioned list of the nonionic surfactant.

(*): The HLB value was adjusted using the two nonionic surfactants having different HLB values in the indicated ratio.

It is apparent from the results given in Table 2 that the spread area of the aqueous albumin solution (aqueous liquid sample) was well controlled to give a very small area in the use of the integral multilayer

EP 0 162 301 B1

analytical element of the invention having a woven fabric spreading layer containing a nonionic surfactant of an HLB value of not less than 10 and a hydrophilic cellulose derivative.

Example 4 & Comparison Example 4

An aqueous gelatin (alkali-treated and deionized) solution was coated on a gelatin subbing layer of a colorless, transparent PET film (support, thickness: 180 $\mu$m) to give a layer of 20 $\mu$m thick (dry basis). The coated layer was dried to give a water-absorbing layer.

One surface of a plain weave broad cloth (thickness: 140 $\mu$m) of polyethylene terephthalate (PET) spun yarn (count: equivalent to approx. cotton count 100 S) was processed by glow discharge for 60 s (1.6 kW/m$^2$, oxygen concentration was controlled under reduced pressure).

On the water-absorbing layer having been almost uniformly wetted with water was placed the processed broad cloth to face the glow discharge-processed surface with the water-absorbing layer. The composite was then passed between pressure rollers to uniformly combine the plain weave broad cloth with the water-absorbing layer.

On the laminated woven fabric spreading layer was coated the aqueous solution for treating the spreading layer having the following composition in the amount to give the coated methylcellulose amount indicated in Table 3 per 1 m$^2$.

| Plain Weave Broad Cloth Treating Solution | |
|---|---|
| Methylcellulose [viscosity 5 Pa•s (50 cps) at 20°C], measured on 2 % aqueous solution) | 20 g. |
| Titanium dioxide microparticles (rutile type, particle size 0.25 - 0.40 $\mu$m) | 100 g. |
| Polyoxyethylene octylphenyl ether (containing 30(average) oxyethylene units, HLB: 17.4) | 10 g. |
| Water | 1000 g. |

The coated solution was dried to prepare an integral multilayer analytical element (Example 3, three kinds of elements were prepared).

An integral multilayer analytical element was prepared in the same manner as in Example 4 except that the woven fabric layer was not treated with the plain weave broad cloth treating solution (Example 4).

On each of the woven fabric spreading layer of the multilayer analytical elements (Example 4 and Comparison Example 3) was spotted 10 $\mu\ell$ of a human plasma, and the element was subjected to incubation at 37ºC for 6 min. The spread area was then measured. Further, the background optical density was measured through the support by reflection photometry. This optical density indicates the light-shielding or light-reflecting function (white background function) of the titanium dioxide microparticles dispersed in the hydrophilic cellulose derivative in the woven fabric spreading layer. The measured values are set forth in Table 3.

11

Table 3

| Methylcellulose Coated Amount | Spread Area | Reflection Optical Density |
|---|---|---|
| $0.95 \ g/m^2$ | $110 \ mm^2$ | 0.21 |
| 1.9 | 105 | 0.25 |
| 9.5 | 102 | 0.25 |
| Not-coated(Com.Ex.3) | 155 | 0.54 |

The results given in Table 3 indicate that the spread area of the aqueous liquid sample in the woven fabric spreading layer of the multilayer analytical element of the invention is small over a wide hydrophilic cellulose derivative concentration range and further that the backgroud optical density is also small in the range. In contrast, the woven fabric spreading layer containing a large amount of a nonioinic surfactant having an HLB value of not less than 10 but containing no hydrophilic cellulose derivative (multilayer analytical element of Comparison Example 3) gives a large spread area in excess of approx. 40 to 60 % from a preferred value and further shows practically disadvantageous background optical density.

Example 5 & Comparison Example 4

The procedures of Example 1 were repeated except that the spreading layer was prepared from a plain weave broad cloth (thickness: 180 μm) of cotton spun yarn (cotton count: 80 S, no glow-discharge treatment had been applied) or a plain weave broad cloth (thickness: 180 μm) of PET/cotton(75%/25%) mixed spun yarn (count: equivalent to cotton count 80 S, glow-discharge treatment had been applied). Thus, two integral multilayer analytical elements having different woven fabric spreading layers were prepared (Example 5).

The procedures of Example 5 were repeated except that the woven fabric spreading layer was not impregnated with the combination of a nonionic surfactant of an HLB value of not less than 10 and a hydrophilic cellulose derivative. Thus, two integral multilayer analytical elements having different woven fabric spreading layers were prepared (Comparison Example 5).

On each of the woven fabric spreading layers of the four multilayer analytical elements was spotted 10 μℓ of a human plasma. The spread area and the spreading conditions were then determined. The results are set forth in Table 4.

Table 4

| Woven Fabric | Spreading Layer | | Spread Area | Spreading Conditions |
|---|---|---|---|---|
| Cotton Broad | Example | 5 | 83 mm$^2$ | Uniform |
| | Com. Ex. | 4 | 131 mm$^2$ | Uniform |
| PET/Cotton | Example | 5 | 90 mm$^2$ | Uniform |
| | Com. Ex. | 4 | 155 mm$^2$ | Less Uniform |

The results given in Table 4 indicate that the multilayer analytical element of the present invention gives uniform spreading to the spotted aqueous liquid sample and shows small spreading area regardless of the material of the porous spreading layer, namely, cotton broad cloth or PET/cotton mixed broad cloth. In the multilayer analytical element of Comparison Example 4, the spreading conditions and spread area are almost the same as those given by the multilayer analtyical element of the invention in the case of using a PET/cotton broad cloth as the porous spreading layer material. However, in the case of using a cotton broad cloth as the spreading layer material, the spreading conditions are less uniform, and the spread area exceeds a preferred value by approx. 30 to 50 %.

Example 6 & Comparison Example 5

A reagent layer for glucose determination (thickness: approx. 15 $\mu$m, dry basis) was formed on a gelatin subbing layer of a colorless, transparent PET film support (thickness: 185 $\mu$m) using the following composition.

| Reagent Composition for Glucose Determination | |
|---|---|
| Glucose oxidase | 15,000 IU |
| Peroxidase | 25,000 IU |
| 1,7-Dihydroxynaphthalene | 5 g. |
| 4-Aminoantipyrine | 12 g. |
| Deionized gelatin | 200 g. |
| Polyoxyethylene nonylphenyl ether (containing 10(average) oxyethylene units) | 2 g. |

On the reagent layer was coated an aqueous dispersion containing 0.2 g. of polyoxyethylene nonylphenyl ether (containing 10(average) oxyethylene units), 8 g. of titanium dioxide microparticles (rutile type, particle size: 0.25 - 0.40 $\mu$m), and 1 g. of deionized gelatin. The coated layer was dried to form a light-shielding layer of approx. 5 $\mu$m thick (dry basis).

On the light-shielding layer was coated a solution of 4 g. of deionized gelatin and 0.2 g. of polyoxyethylene nonylphenyl ether (containing 10(average) oxyethylene units) in 100 m$\ell$ of water. The coated layer was dried to form an adhesive layer of approx. 2 $\mu$m thick (dry basis).

One surface of a plain weave broad cloth (thickness: 140 $\mu$m) of PET spun yarn (count: equivalent to cotton count 100 S) was processed by glow discharge for 60 s (1.6 kW/m$^2$, oxygen concentration was controlled under reduced pressure).

On the adhesive layer having been almost uniformly wetted with water was placed the processed broad cloth to face the glow discharge-processed surface with the adhesive layer. The composite was then passed between pressure rollers to uniformly combine the plain weave broad cloth with the adhesive. Thus, a woven fabric spreading layer was formed.

On the spreading layer was coated the woven fabric spreading layer treating solution having the below-stated composition in the amount of 150 mℓ per 1 m². The coated layer was dried to give an integral multilayer analytical element for quantitative determination of glucose concentration in blood (Example 6).

| Woven Fabric Spreading Layer Treating Solution | |
|---|---|
| Methylcellulose [viscosity 5 Pa•s (50 cps) at 20°C], measured on 2 % aqueous solution) | 20 g. |
| Titanium dioxide microparticles (rutile type, particle size: 0.25 - 0.40 μm) | 100 g. |
| Polyoxyethylene octylphenyl ether (containing 30(average) oxyethylene units, HLB: 17.4) | 10 g. |
| Water | 1000 g. |

Separately, an integral multilayer analytical element for quantitative determination of glucose concentration in blood was prepared in the same manner as above (Example 6) except that the porous spreading layer was not treated with the woven fabric spreading layer treating solution (Comparison Example 5).

On each of the integral multilayer analytical elements for quantitative determination of glucose in blood (the elements of Example 6 and Comparison Example 5) was spotted 10 μℓ of a human serum having the glucose content indicated in Table 3 which was prepared by adding glucose to a human serum. The element was subjected to incubation at 37°C for 360 s. The color formation shown on the element was then measured by reflection photometry with a visible ray of 500 nm (central wavelength) using a reflection optical densitometr. The measured values are set forth in Table 5.

Table 5

| Glucose Concent- | Measured Reflection Optical Density | |
|---|---|---|
| ration (mg/dℓ) | Example 6 | Com. Ex. 5 |
| 103 | 0.497 | 0.271 |
| 252 | 0.750 | 0.360 |
| 320 | 0.839 | 0.410 |
| 450 | 0.982 | 0.460 |

| Aqueous Liquid | Spread Area (Measured Value) | |
|---|---|---|
| Sample | Example 6 | Com.Ex. 5 |
| Human Serum | 128 mm² | 270 mm² |
| Versatol-P | 127 mm² | 280 mm² |

Remark: The glocose concentration value of the human serum was obtained by Hexokinase-G-6-PDH method. Versatol-P: control serum, tradename of Werner-Lambert Corp.

The results set forth in Table 5 indicate that the integral multilayer analytical element of the invention

EP 0 162 301 B1

gives a calibration curve having an incline larger than that given by the integral multilayer analytical element of the Comparison Example. Further, the integral multilayer analytical element of the invention gives a calibration curve having a large incline even in the region of high glucose concentration. For the reasons, the analytical element of the invention makes it possible to determine very accurately the glucose content over wide range.

Example 7

One surface of a tricot knitted fabric (thickness: approx. 250 $\mu$m) of PET spun yarn (equivalent to approx. 50 D) having been defatted by washing with water was processed by glow discharge for 60 s (1.6 kW/m$^2$, oxygen concentration was controlled under reduced pressure).

The procedures of Example 6 were repeated except that the spreading layer was prepared from the above-processed knitted fabric. Thus, an integral multilayer analytical element for quantitative determination of glucose.

This element was subjected to the same measurement as in Example 6. The results are set forth in Table 6.

Table 6

| Glucose Concent-ration (mg/dℓ) | Measured Reflection Optical Density | |
| --- | --- | --- |
| | Example 7 | Com. Ex. 5 |
| 103 | 0.640 | 0.271 |
| 252 | 1.005 | 0.360 |
| 320 | 1.142 | 0.410 |
| 450 | 1.353 | 0.460 |

| Aqueous Liquid Sample | Spread Area (Measured Value) | |
| --- | --- | --- |
| | Example 7 | Com.Ex. 5 |
| Human Serum | 110 mm$^2$ | 270 mm$^2$ |
| Versatol-P | 115 mm$^2$ | 280 mm$^2$ |

The results given in Table 6 indicate that the integral multilayer analytical element having a knitted fabric spreading layer (which contains a hydrophilic cellulose derivative and a nonionic surfactant having an HLB of not less than 10) as prepared in the above Example 7 is superior to the integral multilayer analytical element having a woven fabric spreading layer (which contains a hydrophilic cellulose derivative and a nonionic surfactant having an HLB of not less than 10) as prepared in Example 6.

**Claims**

1. An integral multilayer analytical element which comprises:
    a porous spreading layer of woven fabric or knitted fabric, which contains a hydrophilic cellulose derivative and a nonionic surfactant having an HLB value of not less than 10, or a combination of such surfactants,
a water-absorbing layer containing a hydrophilic polymer, and
a light-transmissive, water-impermeable support.

2. The integral multilayer analytical element as claimed in claim 1, wherein said hydrophilic cellulose derivative of the spreading layer is a water-soluble cellulose ether.

3. The integral multilayer analytical element as claimed in claim 1, wherein said hydrophilic cellulose derivative of the spreading layer is methylcellulose, ethylcellulose, hydroxyethylcellulose, hydroxypropylmethylcellulose, or hydroxybutylmethylcellulose.

4. The integral multilayer analytical element as claimed in claim 1, wherein said hydrophilic cellulose derivative is incorporated into the porous spreading layer in an amount of 0.5 to 15 g per 1 $m^2$ of the porous spreading layer.

5. The integral multilayer analytical element as claimed in claim 1, wherein said nonionic surfactant is incorporated into the porous spreading layer in an amount of 0.1 to 3 g per 1 $m^2$ of the porous spreading layer.

6. The integral multilayer analytical element as claimed in claim 1, wherein said water-absorbing layer contains at least one reagent being reactive to an analyte in a liquid sample to be spotted on the spreading layer to show a detectable change and said spreading layer contains titanium dioxide particles.

**Revendications**

1. Elément analytique multicouche intégral, comprenant :
une couche totalement poreuse en étoffe tissée ou en étoffe tricotée, contenant un dérivé cellulosique hydrophile et un surfactant non ionique ayant un chiffre d'équilibre hydrophile-lipophile supérieur ou égal à 10, ou une association de tels surfactants,
une couche absorbant l'eau, contenant un polymère hydrophile, et
un support imperméable à l'eau, translucide.

2. Elément analytique multicouche intégral selon la revendication 1, dans lequel ledit dérivé cellulosique hydrophile de la couche d'étalement est un éther cellulosique hydrosoluble.

3. Elément analytique multicouche intégral selon la revendication 1, dans lequel ledit dérivé cellulosique hydrophile de la couche d'étalement est de la méthylcellulose, de l'éthylcellulose, de l'hydroxyéthylcellulose, de l'hydroxypropylméthylcellulose ou de l'hydroxybutylméthylcellulose.

4. Elément analytique multicouche intégral selon la revendication 1, dans lequel ledit dérivé cellulosique hydrophile est incorporé dans la couche d'étalement poreuse en une quantité comprise entre 0,5 et 15 $g/m^2$ de couche d'étalement poreuse.

5. Elément analytique multicouche intégral selon la revendication 1, dans lequel ledit surfactant non ionique est incorporé dans la couche d'étalement poreuse en une quantité comprise entre 0,1 et 3 $g/m^2$ de couche d'étalement poreuse.

6. Elément analytique multicouche intégral selon la revendication 1, dans lequel ladite couche absorbant l'eau contient au moins un réactif réagissant à une substance d'un échantillon liquide à détecter sur la couche d'étalement, pour produire une modification détectable, et ladite couche d'étalement contient des particules de dioxyde de titane.

**Patentansprüche**

1. Integriertes vielschichtiges analytisches Element, dadurch **gekennzeichnet,** daß es umfaßt:
eine poröse Ausbreitungsschicht aus einem gewebten Flächengebilde bzw. Textilmaterial oder einer Maschenware, die ein hydrophiles Cellulosederivat und ein nichtionisches oberflächenaktives Mittel mit einem HLB-Wert von nicht weniger als 10 oder ein Gemisch solcher oberflächenaktiver Mittel enthält;

eine Wasserabsorptionsschicht, die ein hydrophiles Polymeres enthält; und

einen lichtdurchlässigen wasserimpermeablen Träger.

2. Integriertes vielschichtiges analytisches Element nach Anspruch 1, dadurch **gekennzeichnet,** daß das hydrophile Cellulosederivat der Ausbreitungsschicht ein wasserlöslicher Celluloseether ist.

3. Integriertes vielschichtiges analytisches Element nach Anspruch 1, dadurch **gekennzeichnet,** daß das hydrophile Cellulosederivat der Ausbreitungsschicht Methylcellulose, Ethylcellulose, Hydroxyethylcellulose, Hydroxypropylmethylcellulose oder Hydroxybutylmethylcellulose ist.

4. Integriertes vielschichtiges analytisches Element nach Anspruch 1, dadurch **gekennzeichnet,** daß das hydrophile Cellulosederivat in die poröse Ausbreitungsschicht in einer Menge von 0,5 bis 15 g pro 1 m$^2$ der porösen Ausbreitungsschicht eingearbeitet ist.

5. Integriertes vielschichtiges analytisches Element nach Anspruch 1, dadurch **gekennzeichnet,** daß das nichtionische oberflächenaktive Mittel in die poröse Ausbreitungsschicht in einer Menge von 0,1 bis 3 g pro 1 m$^2$ der porösen Ausbreitungsschicht eingearbeitet ist.

6. Integriertes vielschichtiges analytisches Element nach Anspruch 1, dadurch **gekennzeichnet,** daß die Wasserabsorptionsschicht mindestens ein Reagens enthält, das gegenüber dem Analyten einer flüssigen Probe, die auf die Ausbreitungsschicht aufgetragen wird, reaktiv ist, wodurch eine nachweisbare Änderung auftritt, und daß die Ausbreitungsschicht Titandioxid-Teilchen enthält.